(19) Europäisches Patentamt
European Patent Office
Office européen des brevets

(11) Veröffentlichungsnummer : **0 098 562**
**B1**

(12) # EUROPÄISCHE PATENTSCHRIFT

(45) Veröffentlichungstag der Patentschrift : **05.03.86**

(51) Int. Cl.[4] : **G 01 N 33/72**

(21) Anmeldenummer : **83106467.0**

(22) Anmeldetag : **02.07.83**

(54) **Verfahren zur Bestimmung von direktem und Gesamt-Bilirubin sowie hierfür geeignetes Reagens.**

(30) Priorität : **07.07.82 DE 3225331**

(43) Veröffentlichungstag der Anmeldung : **18.01.84 Patentblatt 84/03**

(45) Bekanntmachung des Hinweises auf die Patenterteilung : **05.03.86 Patentblatt 86/10**

(84) Benannte Vertragsstaaten : **AT BE CH DE FR GB IT LI LU NL SE**

(56) Entgegenhaltungen :
**EP-A- 0 022 240**
**EP-A- 0 022 673**
**EP-A- 0 028 123**
**US-A- 4 338 095**
**Z. klin. Chem. u. klin. Biochem. 1971 (3), S. 273-274, G. MILLMANN**

(73) Patentinhaber : **Boehringer Mannheim GmbH**
**Sandhofer Strasse 116**
**D-6800 Mannheim 31 (DE)**

(72) Erfinder : **Weiss, Ludwig, Dr.med.**
**Hauberrisserstrasse 7**
**D-8000 München 90 (DE)**
Erfinder : **Hoffmann, Georg-Erich, Dr.med.**
**Hubertusstrasse 29**
**D-8082 Grafrath (DE)**

**EP 0 098 562 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Bilirubin kommt im menschlichen Organismus hauptsächlich als wichtigstes Abbauprodukt von Hämoglobin vor. In der Leber wird Bilirubin teilweise mit Glucuronsäure konjugiert. Das glucuronierte Bilirubin wird als « direktes » Bilirubin bezeichnet. Der mit Glucuronsäure nicht konjugierte Teil wird « indirektes » Bilirubin genannt. Normalerweise befinden sich nur geringe Mengen Bilirubin im Blut. Die Normalkonzentrationen betragen :

für direktes Bilirubin : bis 0,25 mg/100 ml Serum (≈ 4,3 μ mol/l),

für indirektes Bilirubin : bis 0,75 mg/100 ml Serum (≈ 12,7 μ mol/l).

Beim gesunden Organismus wird Bilirubin von der Galle mit der Gallenflüssigkeit in den Darm ausgeschieden. Dieser Mechanismus ist bei verschiedenen Krankheiten gestört. So kann beispielsweise bei vermehrtem Hämoglobinzerfall das Glucuronidierungssystem überlastet werden, so daß sich das Verhältnis direktes/indirektes Bilirubin ändert. Auch bei Neugeborenen ist die Glucuronidierungs-Kapazität unterentwickelt, was ebenfalls zu Verschiebungen im Verhältnis zwischen direktem und indirektem Bilirubin führt. Bei Leberzellschädigungen, Störungen des Ausflusses in die Gallenkapillare oder bei Gallengangverschlüssen ist die Ausscheidung des Bilirubins über die Galle in den Darm vermindert oder völlig blockiert. Dies führt zu erhöhten Bilirubinkonzentrationen im Blut. Dabei kann sowohl die absolute Konzentration des Bilirubins als auch das Verhältnis direktes/indirektes Bilirubin beeinflußt werden. Aus der Messung beider Werte lassen sich somit wichtige diagnostische Schlüsse auf Art und Lokalisation bestimmter Erkrankungen des Leber-Gallen-Darm-Traktes ziehen. Die Bestimmung beider Bilirubin-Fraktionen hat daher in der medizinischen Diagnostik eine besondere Bedeutung erlangt. Hierzu wird üblicherweise zunächst das Gesamt-Bilirubin bestimmt. Danach wird der direkte Bilirubingehalt gemessen. Der indirekte Bilirubinanteil ergibt sich aus der Differenz beider Werte.

Die am häufigsten ausgeführten Bilirubin-Nachweisreaktionen beruhen auf der Kupplung des Bilirubins mit Diazoniumsalzen. Nach Jendrassik und Cleghorn (Biochem. Z. *289* (1937), S. 1) wird zur Bestimmung des direkten Bilirubins 1 ml Serum mit 0,5 ml einer Diazomischung versetzt, die aus 5 g Sulfanilsäure, 15 ml konzentrierter Salzsäure und 0,25 ml 0,5 % iger Natriumnitrit-Lösung in Wasser besteht. Besonders nachteilig ist bei dieser Bestimmungsmethode, daß die Reaktion in stark saurem Milieu durchgeführt werden muß.

Zur Bestimmung von Gesamt-Bilirubin wird der Probe zunächst Coffein und Natrium-Benzoat zugesetzt und erst anschließend mit diazotierter Sulfanilsäure gekuppelt. Der Zusatz von Coffein und Natrium-Benzoat hat den Zweck, indirektes Bilirubin aus seiner Bindung an Albumin zu lösen und Störungen durch einen ohne diese Zusätze auftretenden Eiweißniederschlag auszuschließen.

Mit diesen Zusatzstoffen kann die Bestimmungsreaktion jedoch nicht im stark sauren Milieu, wie bei der Bestimmung des direkten Bilirubins, durchgeführt werden, sondern es muß im neutralen bzw. schwach sauren Bereich gearbeitet werden. Diese Reaktionsbedingungen haben jedoch den Nachteil, daß dabei mit anderen Inhaltsstoffen der Probe (beispielsweise mit Phenolen) bräunlich gefärbte Nebenprodukte entstehen, die den Test in zahlreichen Fällen stören. Auch die Reagenslösung selbst ist nicht über längere Zeit stabil. Auch ohne Probe entsteht eine Substanz, die zwar im sauren Milieu farblos, aber bereits im neutralen Bereich gelb und im alkalischen rot ist. Um Fehler, die aufgrund dieser Störungen auftreten können, kleinzuhalten, werden zuweilen sehr komplizierte Schritte vorgeschlagen. Beispielsweise arbeitet man nach Jendrassik und Cleghorn bei zwei verschiedenen Wellenlängen oder es wird nach Abschluß der Diazotierungsreaktion im schwach sauren bzw. neutralen Bereich vor der eigentlichen Messung der Testansatz ins alkalische Milieu übergeführt (Jendrassik und Grof, Biochem. Z. *297* (1938) Seite 81-89).

All diese Bestimmungsmethoden sind in der Ausführung unangenehm und unpraktisch. Darüberhinaus sind sie wegen der zahlreichen Schritte immer noch mit großen Ungenauigkeiten und Fehlern behaftet. Es hat daher nicht an Versuchen gefehlt, die Bestimmungsmethoden für direktes und Gesamt-Bilirubin zu verbessern.

Es wurde z. B. versucht, auch die Bestimmung des Gesamt-Bilirubins in das stark saure Milieu zu verlagern. Hierzu war es vor allem erforderlich, nach Hilfsstoffen zu suchen, die im stark sauren Milieu die Proteinbindung aufzubrechen im Stande sind. So werden beispielsweise von Bartels und Boehmer, Z. Clin. Chem. Clin. Biochem. *7* (1969), S. 444 Dispersionsmittel und von Winsten und Cehelyk, Clin. Chem. Acta *25* (1969), S. 441 Dimethylsulfoxyd als « Lösungsvermittler » für das indirekte Bilirubin eingesetzt, um anschließend das Gesamt-Bilirubin in stark saurem Milieu in analoger Weise zur Bestimmung des direkten Bilirubins durchführen zu können.

Trotz der zahlreichen Varianten können die in der Literatur bisher beschriebenen Testmethoden für direktes und Gesamt-Bilirubin die Fachwelt immer noch nicht befriedigen, wie die schlechte Übereinstimmung verschiedener Methoden zeigt (Jacobs et al., Clin. Chem. *10* (1964) S. 433-439). Vor allem das stark saure, aggressive Milieu, in dem die bisher bekannten Bestimmungsmethoden durchgeführt werden müssen, um eine einigermaßen praktikable Differenzierung zu erreichen (loc. cit.), sind für die praktische Durchführung der Teste unvorteilhaft.

Aufgabe der vorliegenden Erfindung war es daher, eine Bestimmungsmethode für direktes und für Gesamt-Bilirubin bereitzustellen, die im schwach sauren bis ungefähr neutralen pH-Bereich durchgeführt werden kann, einfach und leicht durchzuführen ist und zuverlässige Werte liefert.

Überraschenderweise wurde gefunden, daß die Bestimmung des direkten Bilirubins und des Gesamt-Bilirubins im schwach sauren bis annähernd neutralen Bereich möglich ist, wenn zur Kopplung mit dem Bilirubin in bestimmter Weise substituierte Diazoniumsalze, beispielsweise diazotiertes 2-Amino-5-chlor-toluol (Fast Red TR) oder diazotiertes 2-Methoxy-5-chlor-anilin (Fast Red RC), verwendet werden.

Gegenstand der Erfindung ist daher ein Verfahren zur Bestimmung des direkten und des Gesamt-Bilirubins in Körperflüssigkeiten, indem das Bilirubin mit einem Diazoniumsalz gekuppelt wird und die dadurch bewirkte Extinktionsänderung nach bekannten Methoden gemessen wird, dadurch gekennzeichnet, daß die Kupplung in Gegenwart eines Puffers, der die Testlösung auf pH 3,5 bis 8 einstellt, durchgeführt und als Kupplungskomponente ein Diazoniumsalz der allgemeinen Formel I

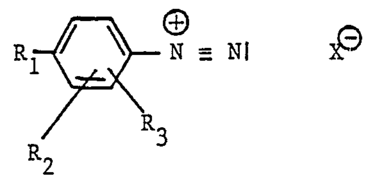

(I)

in der

$R_1$ Wasserstoff, Halogen, eine niedere Alkyl-, eine niedere Alkoxy- oder eine Benzoylamino-Gruppe,

$R_2$ Wasserstoff, eine niedere Alkyl- oder niedere Alkoxy-Gruppe,

$R_3$ Wasserstoff, Halogen, eine niedere Alkyl- oder eine niedere Alkoxy-Gruppe, wobei $R_1$ und $R_3$ nicht gleichzeitig Halogen darstellen dürfen, und

X ein mit dem Diazoniumkation ein lagerstabiles Salz bildendes Anion,

bedeuten,

verwendet wird.

Als niedere Alkyl- bzw. niedere Alkoxygruppen in der Definition der Substituenten $R_1$, $R_2$ und $R_3$ werden Reste mit 1-5, vorzugsweise 1-3 C-Atomen verstanden. Besonders bevorzugt sind die Methyl- und Ethyl- bzw. die Methoxy- und Ethoxy-Gruppe.

Unter Halogen sind Fluor, Chlor, Brom und Jod zu verstehen, wobei Chlor und Brom besonders bevorzugt sind.

Als ein mit dem Diazoniumkation ein lagerstabiles Salz bildendes Anion kommt vorzugsweise Tetrafluoro-borat oder das Tetrachloro-zinkat infrage.

Die erfindungsgemäß verwendeten Diazoniumsalze sind bekannte Verbindungen oder können in Analogie zu bekannten Verbindungen leicht hergestellt werden. Teilweise sind diese Diazoniumsalze auch schon zur Bestimmung von Bilirubin in stark saurem Medium eingesetzt worden. Für den Fachmann war es jedoch überraschend, daß zwischen Bilirubin und den Diazoniumsalzen der allgemeinen Formel I auch in schwach saurem bis annähernd neutralem Milieu eine Kupplung stattfindet. In Z. Clin. Chem. Clin. Biochem. *9* (1971), S. 273 wird beispielsweise ausdrücklich erwähnt, daß ein im Sinne der vorliegenden Erfindung besonders bevorzugtes Diazoniumsalz, das diazotierte 2-Amino-5-chlor-toluol (Fast Red TR) mit Bilirubin bei einem pH-Wert 5,2 nicht reagiert.

In EP-A 0 021 598 wird ein Verfahren zur kinetischen Bestimmung des Gesamt-Bilirubins beschrieben, bei dem Diazoniumsalze verwendet werden, die teilweise den erfindungsgemäß zu verwendenden Verbindungen der allgemeinen Formel I entsprechen. Zur Durchführung der kinetischen Messung ist es jedoch erforderlich, Diazoniumsalze zusammen mit reaktionsbeschleunigenden Mitteln einzusetzen.

Ein weiterer Gegenstand der Erfindung ist ein Mittel zur Durchführung des erfindungsgemäßen Verfahrens zur Bestimmung von direktem und Gesamt-Bilirubin, das die zur Bestimmung erforderlichen Bestandteile, Diazoniumsalz und Puffer, in Form einer Lösung, einer Pulvermischung, einer Reagenstablette, eines Lyophilisates oder eines damit imprägnierten saugfähigen Trägers enthält.

Zur Bestimmung des Gesamt-Bilirubins und des direkten Bilirubins kann das Mittel zusätzlich einen Lösungsvermittler enthalten.

Erfindungsgemäß wird ein Puffer eingesetzt, der die Testlösung auf einen pH-Wert im Bereich von 3,5-8,0 einstellt. Zur Bestimmung des direkten Bilirubins wird vorzugsweise ein Puffer eingesetzt, der in dem pH-Bereich 3,5-6,0, vorzugsweise 4,3-5,7, wirksam ist. Für die Bestimmung des Gesamt-Bilirubins wird vorzugsweise ein in dem pH-Bereich von 5,5-8, vorzugsweise 6-7, wirksamer Puffer verwendet.

Als Puffer können solche verwendet werden, die im erwähnten pH-Bereich hinreichende Pufferkapazität besitzen. Für die Bestimmung von direktem Bilirubin haben sich besonders Puffersysteme wie beispielsweise Citronensäure/Tris-(hydroxymethyl)-aminomethan, Citronensäure/Natronlauge, Essigsäure/Natronlauge, Kaliumhydrogen-Phthalat/Natronlauge oder Phosphatpuffer erwiesen, wobei die drei erstgenannten Puffersysteme ganz besonders bevorzugt sind. Für die Bestimmung von Gesamt-Bilirubin sind besonders bevorzugt Citronensäure/Tris-(hydroxymethyl)-aminomethan, Salzsäure/Tris-(hydroxymethyl)-aminomethan. Es kann auch Salzsäure/Imidazol-Puffer verwendet werden.

Zur Bestimmung des Gesamt-Bilirubins und des direkten Bilirubins kann zusätzlich ein Löslichkeitsvermittler zugesetzt werden. Zu diesem Zweck haben sich als besonders geeignet erwiesen Dimethylformamid, Dimethylsulfoxyd, Tetrahydrofuran, Dioxan oder verschiedene Glykole. Dimethylformamid ist besonders bevorzugt. Zur Bestimmung des direkten Bilirubins kann ein nicht-ionisches Detergens, vorzugsweise Triton X 100® (Rohm & Haas, Philadelphia) zugesetzt werden, wobei die Konzentration an Triton X 100® etwa 0,1-1,0 % beträgt. Weitere Beispiele für nichtionische Detergentien sind Tween 21® oder Tween 40® (Hersteller ICI), Tergitol® 15-S-12 (Union Carbide Corp.), BRIJ 78 (ICI).

Zur Bestimmung von direktem Bilirubin und von Gesamt-Bilirubin wird die zu bestimmende Probe mit dem Diazoniumsalz, dem Puffer und gegebenenfalls mit dem Lösungsvermittler in Kontakt gebracht. Die zugesetzten Stoffe können dabei als Lösung, als Pulvermischung, als Reagenstablette, als Lyophilisat oder in Form eines damit imprägnierten saugfähigen Trägers vorliegen. Es ist möglich, die verschiedenen Formen zu kombinieren. So kann beispielsweise der Puffer als Lösung in einem geeigneten Lösungsmittel, das Diazoniumsalz in Form einer Tablette eingesetzt werden.

Die Konzentration des zu messenden Bilirubins im Testansatz sollte zwischen 0,5 und 25 μmol/l liegen. Gegebenenfalls ist die Ausgangsprobe entsprechend zu verdünnen.

Die eingesetzte Menge an Diazoniumsalz, Puffer und gegebenenfalls Lösungsvermittler wird so gewählt, daß in der fertigen Testlösung folgende Konzentrationen erreicht werden :

Diazoniumsalz : zwischen 0,5 und 30 mmol/l, vorzugsweise zwischen 5 und 15 mmol/l,

Puffer : zwischen 0,02 und 1,0 mol/l, vorzugsweise 0,05 und 0,3 mol/l,

gegebenenfalls zugesetzter Lösungsvermittler : zwischen 0,1 und 4 mol/l, vorzugsweise zwischen 1 und 3 mol/l bei Messung des Gesamt-Bilirubins und 0,01-0,1 mol/l bei Messung des direkten Bilirubins.

Ein wesentlicher Vorteil des erfindungsgemäßen Verfahrens zur Bestimmung von direktem und Gesamt-Bilirubin liegt darin, daß sich die jeweiligen Reaktionsbedingungen nur durch geringfügige Unterschiede im pH-Wert der Testlösung unterscheiden. Beide Bestimmungen können somit nebeneinander mit weitgehend identischen Reagentien durchgeführt werden. Vorteilhafterweise wird zu Bestimmung des direkten und des Gesamt-Bilirubins dasselbe diazoniumsalz verwendet. Der erforderliche unterschiedliche pH-Wert wird durch geringe Variation in der Zusammensezung der den pH-Wert bestimmenden Puffersubstanzen erreicht.

Praktisch kann so vorgegangen werden, daß für die Bestimmung des direkten und des Gesamt-Bilirubins jeweils getrennte Reagentienzusammensetzungen bereitgestellt werden, die dasselbe Diazoniumsalz enthalten, jedoch unterschiedliche Zusammensetzung der Pufferkomponenten aufweisen. Es kann auch so vorgegangen werden, daß für beide Bestimmungen eine gemeinsame Reagentienkombination bereitet wird, die das Diazoniumsalz und einen Puffer zur Einstellung des pH-Wertes für den einen Bestimmungsparameter enthält. Der pH-Wert für die Bestimmung des anderen Parameters wird durch den Zusatz weiterer geeigneter Puffer-Substanzen eingestellt.

Das erfindungsgemäße Mittel zur Bestimmung von direktem oder Gesamt-Bilirubin in Form einer Lösung enthält vorzugsweise sämtliche für den Test benötigten Reagentien. Als Lösungsmittel kommen Wasser oder Gemische von Wasser mit einem wasserlöslichen organischen Lösungsmittel, wie z. B. Methanol, Ethanol, Aceton oder Dimethylformamid, infrage. Aus Haltbarkeitsgründen kann es vorteilhaft sein, die für den Test benötigten Reagentien auf zwei oder mehr Lösungen zu verteilen, die erst bei der eigentlichen Bestimmung zusammengegeben werden.

Das erfindungsgemäße Mittel in Form von Pulvermischungen oder einer Reagenstablette läßt sich herstellen, indem die erforderlichen Bestandteile des Testes mit üblichen galenischen Zusatzstoffen versetzt und granuliert werden. Zusatzstoffe dieser Art sind z. B. Kohlenhydrate, wie z. B. Mono-, Oligo- oder Polysaccharide, oder Zuckeralkohole, wie z. B. Mannit, Sorbit oder Xylit, oder andere lösliche inerte Verbindungen, wie Polyethylenglykole oder Polyvinylpyrrolidon. Die Reagenstabletten weisen im allgemeinen ein Endgewicht von ungefähr 20-200 mg, vorzugsweise 50-80 mg, auf.

Zur Herstellung von Lyophilisaten wird eine Lösung gefriergetrocknet, die neben sämtlichen für die Bestimmung benötigten Reagentien üblicher Gerüstbildner, wie z. B. Polyvinylpyrrolidon, und eventuell weitere Füllstoffe, wie z. B. Mannit, Sorbit oder Xylit, enthält. Zur Herstellung des erfindungsgemäßen Mittels zur Bestimmung von Bilirubin in an einem saugfähigen Träger absorbierter Form wird der saugfähige Träger, vorzugsweise Filterpapier, Zellulose oder Kunstfaservliese, mit Lösungen der erforderlichen Testbestandteile in leicht flüchtigen Lösungsmitteln, wie z. B. Wasser, Methanol, Ethanol oder Aceton, imprägniert. Die fertigen Testpapiere können als solche verwendet werden oder in an sich bekannter Weise an Griffen angeklebt oder vorzugsweise zwischen Kunststoffen und feinmaschigen Netzwerken gemäß DBP 21 18 455 eingesiegelt werden.

Die einzelnen zur Bestimmung von Bilirubin erforderlichen Reagentien können auch auf einen Teststreifen aufgebracht werden. In diesem Falle wird auf das die Reagentienbestandteile enthaltende Testfeld die Bilirubin-haltige Probe, beispielsweise Serum, aufgetropft und anhand der auftretenden Farbänderung in an sich bekannter Weise der Bilirubin-Gehalt der Probe bestimmt.

Die Erfindung wird durch die folgenden Beispiele erläutert :

## Beispiel 1

1. Lösungen :

Puffer-Lösung : 167 mmol Tris-(hydroxymethyl)-aminomethan und 83 mmol Citronensäure werden mit destilliertem Wasser zu einem Liter Gesamt-Volumen gelöst. Die Lösung weist einen pH-Wert von 5,0 auf.

Reaktionslösung 1 : In 10 ml der oben erwähnten Pufferlösung werden 20 mg Fast Red TR (diazotiertes 2-Amino-5-chlor-toluol × 1/2 $ZnCl_2$) gelöst. Die Lösung ist mindestens 24 Std. bei Raumtemperatur haltbar.

Lösung 2 (Zusatzreagens für die Bestimmung von Gesamt-Bilirubin) : 0,9 mol Tris-(hydroxymethyl)-aminomethan werden mit destilliertem Wasser zu einem Liter Gesamt-Volumen gelöst.

2. Testdurchführung :

In Mikroküvetten (Schichtdicke 1 cm) werden bei Raumtemperatur die verschiedenen Reagentienlösungen in der folgenden Weise pipettiert :

| | A = direktes Bilirubin | | B = Gesamt-Bilirubin | |
|---|---|---|---|---|
| | Meßwert | Leerwert | Meßwert | Leerwert |
| Reaktionslsg. 1 | 500 µl | — | 500 µl | — |
| Puffer | — | 500 µl | — | 500 µl |
| dest. Wasser | 50 µl | 50 µl | — | — |
| Lösung 2 | — | — | 50 µl | 50 µl |
| Messung d. Extinktion bei 546 nm ($E_1$) | | | | |
| Serum | 20 µl | 20 µl | 20 µl | 20 µl |
| Messung der Extinktion nach genau 10 min ($E_2$) bei 546 nm. | | | | |

Für jede Testserie wird ein Reagentienleerwert ermittelt. Dieser Wert wird erhalten, indem man in analoger Weise vorgeht, wie oben unter « Meßwert » angegeben, jedoch wird das Serum durch die gleiche Menge Wasser ersetzt.

3. Auswertung :

Aus den gemessenen Extinktionen werden die Extinktionsdifferenzen wie folgt berechnet :

$\Delta E_A = (E_2 - E_1)_{A\text{-Meßküvette}} - (E_2 - E_1)_{A\text{-Leerwertküvette}} - (E_2 - E_1)_{A\text{-Reagentienleerwert}}$

$\Delta E_B = (E_2 - E_1)_{B\text{-Meßküvette}} - (E_2 - E_1)_{B\text{-Leerwertküvette}} - (E_2 - E_1)_{B\text{-Reagentienleerwert}}$

Aus den erhaltenen Extinktionsdifferenzen wird der Bilirubin-Gehalt der Probe wie folgt ermittelt :

$\Delta E_A \times 0{,}93$ = mmol direktes Bilirubin/l

$\Delta E_B \times 0{,}93$ = mmol Gesamt-Bilirubin/l.

## Beispiel 2

1. Lösungen :

Pufferlösung A (für direktes Bilirubin) : Eine Lösung von 0,13 mol Citronensäure in Wasser wird mit 0,35 mol Tris-(hydroxymethyl)-aminomethan versetzt und mit Wasser auf 1 Liter Gesamt-Volumen aufgefüllt. Die Lösung weist einen pH-Wert von 5,7 auf.

Pufferlösung B (für Gesamt-Bilirubin) : Eine Lösung von 0,2 mol Tris-(hydroxymethyl)-aminomethan in Wasser wird mit Salzsaüre auf pH 7,0 gebracht. Es werden 50 ml Dimethylformamid zugefügt und mit destilliertem Wasser auf 1 Liter Gesamt-Volumen aufgefüllt.

Reagenslösung A bzw. B : Zu je 10 ml Puffer A bzw. B werden 40 mg Fast Violet B (diazotiertes 6-Benzoyl-amino-4-methoxy-3-amino-toluol × 1/2 $ZnCl_2$) gelöst.

2. Testdurchführung :

In Küvetten (Schichtdicke 1 cm) werden bei Raumtemperatur die unten angegebenen Lösungen wie folgt pipettiert :

(Siehe Tabelle Seite 6 f.)

| | A = direktes Bilirubin | B = Gesamt-Bilirubin |
|---|---|---|
| | Meßwert | Meßwert |
| Reagenslsg. A<br>Reagenslsg. B | 2 ml<br>— | —<br>2 ml |
| Messung der Extinktion bei 578 nm ($E_1$) | | |
| Serum | 0,08 ml | 0,08 ml |
| Messung der Extinktion nach genau 10 min ($E_2$) bei 578 nm | | |

Für jede Testserie wird ein Reagentienleerwert ermittelt. Dieser Wert wird erhalten, indem in analoger Weise wie oben unter « Meßwert » angegeben, vorgegangen wird, jedoch wird Serum durch die gleiche Menge Puffer A bzw. B ersetzt.

Eine Leerwertküvette entsprechend Beispiel 1, bei der anstelle der Reagenslösung die entsprechende Menge Pufferlösung eingesetzt wird, ist nur bei deutlich trüben bzw. hämolytischen Seren erforderlich. .

3. Auswertung :

Aus den gemessenen Extinktionen werden Extinktionsdifferenzen wie folgt berechnet :

$\Delta E_A = (E_2 - E_1)_{A\text{-Meßküvette}} - (E_2 - E_1)_{A\text{-Reagentienleerwert}}$

$\Delta E_B = (E_2 - E_1)_{B\text{-Meßküvette}} - (E_2 - E_1)_{B\text{-Reagentienleerwert}}$

Aus den erhaltenen Extinktionsdifferenzen wird der Bilirubin-Gehalt in der Probe wie folgt ermittelt :

$\Delta E_A \times 0{,}49$ = mmol direktes Bilirubin/l

$\Delta E_B \times 0{,}49$ = mmol Gesamt-Bilirubin/l.

Alternativ kann die Bilirubin-Bestimmung auch dadurch erfolgen, daß die Extinktionsdifferenz zwischen der ersten und fünften Minute nach dem Start abgelesen wird (kinetische Messung).

Der Bilirubin-Gehalt in der Probe kann auch auf die Weise bestimmt werden, daß durch Messen verschiedener Serum-Proben mit bestimmtem, bekanntem Bilirubin-Gehalt eine Eichkurve aufgestellt wird, aus der dann für jede Extinktionsdifferenz, die bei der Messung einer Probe mit unbekanntem Bilirubin-Gehalt ermittelt worden ist, die entsprechende Bilirubin-Konzentration abgelesen werden kann.

Gleiche Werte für direktes Bilirubin wie vorstehend werden erhalten, wenn man statt Pufferlösung A einen Citratpuffer mit Zusatz von Triton X 100® verwendet.

## Beispiel 3

1. Lösungen :

Pufferlösung : 73 mmol Tris-(hydroxymethyl)-aminomethan und 49 mmol Citronensäure werden mit destilliertem Wasser gelöst und zu einem Liter Gesamt-Volumen aufgefüllt. Die Lösung weist einen pH-Wert von 4,3 auf.

Reaktionslösung 1 : In 10 ml dieses Puffers werden 20 mg Fast Red RC (diazotiertes 2-Methoxy-5-chloranilin × 1/2 $ZnCl_2$) gelöst. Die Lösung ist mindestens 24 Std. bei Raumtemperatur haltbar.

Lösung 2 (Zusatzreagens für Gesamt-Bilirubin) : 0,75 mol Tris-(hydroxymethyl)-aminomethan werden mit destilliertem Wasser auf 1 Liter aufgefüllt.

2. Testdurchführung :

In Mikroküvetten (Schichtdicke 1 cm) werden bei Raumtemperatur die nachfolgenden Lösungen in der angegebenen Weise pipettiert :

(Siehe Tabelle Seite 7 f.)

| | A = direktes Bilirubin | | B = Gesamt-Bilirubin | |
|---|---|---|---|---|
| | Meßwert | Leerwert | Meßwert | Leerwert |
| Reaktionslsg. 1 | 500 µl | — | 500 µl | — |
| Puffer | — | 500 µl | — | 500 µl |
| dest. Wasser | 50 µl | 50 µl | — | — |
| Lösung 2 | — | — | 50 µl | 50 µl |
| Messung der Extinktion bei 546 nm ($E_1$) | | | | |
| Serum | 20 µl | 20 µl | 20 µl | 20 µl |
| Messung der Extinktion nach genau 10 min bei 546 nm ($E_2$) | | | | |

Für jede Testserie wird ein Reagentienleerwert ermittelt. Dieser wird erhalten, indem in analoger Weise wie oben unter « Meßwert » angegeben, verfahren wird, jedoch anstelle von Serum die gleiche Wassermenge eingesetzt wird.

3. Auswertung :

Aus den gemessenen Extinktionen werden die Extinktionsdifferenzen wie folgt berechnet :
$\Delta E_A = (E_2 - E_1)_{A\text{-Meßküvette}} - (E_2 - E_1)_{A\text{-Leerwertküvette}} - (E_2 - E_1)_{A\text{-Reagentienleerwert}}$
$\Delta E_B = (E_2 - E_1)_{B\text{-Meßküvette}} - (E_2 - E_1)_{B\text{-Leerwertküvette}} - (E_2 - E_1)_{B\text{-Reagentienleerwert}}$
Aus den erhaltenen Extinktionsdifferenzen läßt sich der Bilirubin-Gehalt der Probe wie folgt ermitteln :
$\Delta E_A \times 0{,}83$ = mmol direktes Bilirubin/l
$\Delta E_B \times 0{,}83$ = mmol Gesamt-Bilirubin/l.
Vergleichbare Resultate werden erhalten, wenn in der o.a. Reaktionslösung 1 20 mg Fast Red RC durch 40 mg Fast Blue BB (diazotiertes 2,5-Diethoxy-4-benzoyl-amino-anilin × 1/2 $ZnCl_2$) oder 40 mg Fast Blue RR (diazotiertes 2,5-Dimethoxy-4-benzoylamino-anilin × 1/2 $ZnCl_2$) eingesetzt werden.

## Beispiel 4

Filterpapier (beispielsweise VS 532 der Fa. Binzer) wird
A mit einer Lösung, die 0,13 mol Citronensäure, 0,35 mol Tris-(hydroxymethyl)-aminomethan und 4,0 g Fast Violet B in einem Liter destilliertem Wasser enthält,
B mit einer Lösung, die 0,2 mol Tris-(hydroxymethyl)-aminomethan, 5 % Triton X 100® und 4,0 g Fast Violet B in einem Liter destilliertem Wasser enthält und mit Salzsäure auf pH 7,0 eingestellt worden ist, imprägniert und bei 30 °C getrocknet.

Zur Bestimmung von direktem Bilirubin wird auf den Teststreifen A und zur Bestimmung von Gesamt-Bilirubin wird auf den Teststreifen B jeweils ein Tropfen Serum gegeben. Nach ca. 5-10 min kann eine Farbänderung in das bräunlich-violette beobachtet werden. Aus der Farbänderung wird durch Vergleich mit einer Farbskala, die mit Hilfe von Serumproben mit bekanntem Bilirubin-Gehalt ermittelt worden ist, der Gehalt der Probe an Bilirubin ermittelt. Der Farbumschlag kann auch mit Hilfe eines Reflektionsphotometers durch Messen der Extinktionsänderung bei 578 nm ermittelt werden.

Ähnliche Ergebnisse werden erhalten, wenn man Filterpapiere mit Lösungen imprägniert, die anstelle des o.a. Fast Violet B Fast Red TR, Fast Red RC, Fast Blue BB oder Fast Blue RR enthalten.

**Patentansprüche**

1. Verfahren zur Bestimmung von direktem und Gesamt-Bilirubin in Körperflüssigkeiten, indem das Bilirubin mit einem Diazoniumsalz gekuppelt wird und die dadurch bewirkte Extinktionsänderung nach bekannten Methoden gemessen wird, dadurch gekennzeichnet, daß die Kupplung in Gegenwart eines Puffers, der die Testlösung auf pH 3,5 bis 8 einstellt, durchgeführt und als Kupplungskomponente ein Diazoniumsalz der allgemeinen Formel I

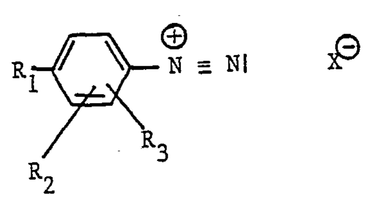

(I)

in der

$R_1$ Wasserstoff, Halogen, eine niedere Alkyl-, eine niedere Alkoxy- oder eine Benzoylamino-Gruppe,
$R_2$ Wasserstoff, eine niedere Alkyl- oder niedere Alkoxy-Gruppe,
$R_3$ Wasserstoff, Halogen, eine niedere Alkyl- oder eine niedere Alkoxy-Gruppe,
wobei $R_1$ und $R_3$ nicht gleichzeitig Halogen darstellen dürfen, und
X ein mit dem Diazoniumkation ein lagerstabiles Salz bildendes Anion,
bedeuten,
verwendet wird.

2. Verfahren gemäß Anspruch 1, dadurch gekennzeichnet, daß das Diazoniumsalz in einer Konzentration von 0,5-30 mmol/l Testlösung und der Puffer in einer Konzentration von 0,02-1 mol/l Testlösung eingesetzt wird.

3. Verfahren gemäß Anspruch 1 oder 2, dadurch gekennzeichnet, daß zur Bestimmung des direkten Bilirubins die Testlösung einen pH-Bereich von 3,5-6,0 aufweist.

4. Verfahren gemäß einem der Ansprüche 1-3, dadurch gekennzeichnet, daß zusätzlich ein lösungsvermittelnder Stoff eingesetzt wird.

5. Verfahren gemäß Anspruch 6, dadurch gekennzeichnet, daß die Konzentration des lösungsvermittelnden Stoffes in der Testlösung bei Bestimmung des Gesamt-Bilirubins 0,1-4 mol/l und bei Bestimmung des direkten Bilirubins 0,01-0,1 mol/l beträgt.

6. Mittel zur Bestimmung des direkten und des Gesamt-Bilirubins in Körperflüssigkeiten nach einem Verfahren gemäß den Ansprüchen 1-5, dadurch gekennzeichnet, daß es ein im pH-Bereich 3,5 bis 8 wirksames Puffersystem und ein Diazoniumsalz der allgemeinen Formel I

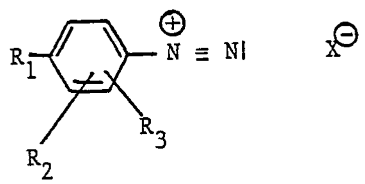

(I)

in der

$R_1$ Wasserstoff, Halogen, eine niedere Alkyl-, eine niedere Alkoxy- oder eine Benzoylamino-Gruppe,
$R_2$ Wasserstoff, eine niedere Alkyl- oder niedere Alkoxy-Gruppe,
$R_3$ Wasserstoff, Halogen, eine niedere Alkyl- oder eine niedere Alkoxy-Gruppe, wobei $R_1$ und $R_3$ nicht gleichzeitig Halogen darstellen dürfen, und
X ein mit dem Diazoniumkation ein lagerstabiles Salz bildendes Anion,
bedeuten,
enthält.

7. Mittel gemäß Anspruch 6, dadurch gekennzeichnet, daß das Diazoniumsalz und der Puffer in einer Konzentration vorliegen, daß in der Testlösung zur Bestimmung des direkten und des Gesamt-Bilirubins eine Endkonzentration von 0,5-30 mmol/l für das Diazoniumsalz und von 0,02-1 mol/l für den Puffer erhalten wird.

8. Mittel gemäß Anspruch 6 oder 7, dadurch gekennzeichnet, daß es zusätzlich ein lösungsvermittelnder Stoff enthält.

9. Mittel gemäß Anspruch 8, dadurch gekennzeichnet, daß der lösungsvermittelnde Stoff in einer Konzentration vorliegt, daß in der Testlösung zur Bestimmung von Gesamt-Bilirubin eine Endkonzentration von 0,1-4 mol/l und zur Bestimmung des direkten Bilirubins 0,01-0,1 mol/l erreicht wird.

**Claims**

1. Process for the determination of direct and total bilirubin in body fluids in which the bilirubin is coupled with a diazonium salt and the extinction change thereby brought about is measured according to known methods, characterised in that the coupling is carried out in the presence of a buffer which adjusts the test solution to pH 3.5 to 8 and, as coupling component, there is used a diazonium salt of the general formula I

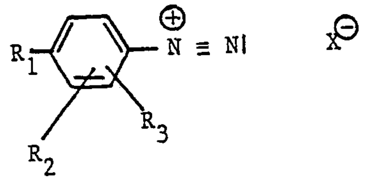

(I)

in which

$R_1$ signifies hydrogen, halogen, a lower alkyl, a lower alkoxy or a benzoylamino group,

$R_2$ hydrogen, a lower alkyl or lower alkoxy group,

$R_3$ hydrogen, halogen, a lower alkyl or a lower alkoxy group, whereby $R_1$ and $R_3$ do not simultaneously represent halogen, and

X is an anion forming a storage-stable salt with the diazonium cation.

2. Process according to claim 1 or 2, characterised in that the diazonium salt is used in a concentration of 0.5-30 mmol/l. of test solution and the buffer in a concentration of 0.02-1 mol/l. of test solution.

3. Process according to claim 1 or 2, characterised in that, for the determination of the direct bilirubin, the test solution has a pH range of 3.5-6.0.

4. Process according to one of claims 1-3, characterised in that a solubilising substance is additionally added.

5. Process according to claim 6, characterised in that the concentration of the solubilising substance in the test solution is, in the case of the determination of total bilirubin, 0.1-4 mol/l. and, in the case of the determination of direct bilirubin, 0.01-0.1 mol/l.

6. Agent for the determination of the direct and of the total bilirubin in body fluids by a process according to any of claims 1-5, characterised in that it contains a buffer system effective in the pH range of 3.5 to 8 and a diazonium salt of the general formula I

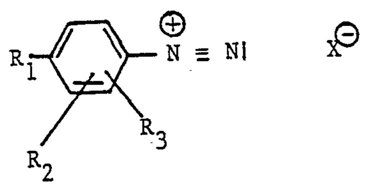

(I)

in which

$R_1$ signifies hydrogen, halogen, a lower alkyl, a lower alkoxy or a benzoylamino group,

$R_2$ hydrogen, a lower alkyl or lower alkoxy group,

$R_3$ hydrogen, halogen, a lower alkyl or a lower alkoxy group, whereby $R_1$ and $R_3$ do not simultaneously represent halogen, and

X is an anion forming a storage-stable salt with the diazonium cation.

7. Agent according to claim 6, characterised in that the diazonium salt and the buffer are present in a concentration such that, in the test solution for the determination of the direct and of the total bilirubin, there is obtained an end concentration of 0.5-30 mmol/l. for the diazonium salt and of 0.02-1 mol/l. for the buffer.

8. Agent according to claim 6 or 7, characterised in that it additionally contains a solubilising substance.

9. Agent according to claim 8, characterised in that the solubilising substance is present in a concentration such that, in the test solution for the determination of total bilirubin, an end concentration of 0.1-4 mol/l. is achieved and, for the determination of the direct bilirubin, of 0.01-0.1 mol/l.

**Revendications**

1. Procédé pour le dosage de la bilirubine directe et de la bilirubine totale dans des liquides physiologiques, en couplant la bilirubine avec un sel de diazonium et en mesurant la variation de l'extinction ainsi provoquée selon une méthode connue, caractérisé en ce que la copulation est effectuée en présence d'un tampon portant la solution à examiner à un pH entre 3,5 et 8, et en ce qu'on utilise comme composant de copulation un sel de diazonium de formule générale I :

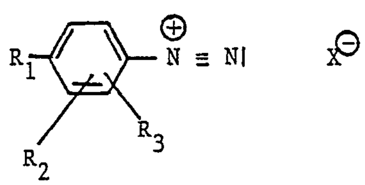

(I)

dans laquelle

$R_1$ est de l'hydrogène, de l'halogène, un groupe alkyle inférieur, alcoxy inférieur ou benzoylamino,

$R_2$ est de l'hydrogène, un groupe alkyle inférieur ou alcoxy inférieur,

$R_3$ est de l'hydrogène, de l'halogène, un groupe alkyle inférieur ou alcoxy inférieur, $R_1$ et $R_3$ ne pouvant pas être en même temps de l'halogène, et

X est un anion formant avec le cation diazonique un sel stable au stockage.

2. Procédé selon la revendication 1, caractérisé en ce que le sel de diazonium est mis en œuvre en une concentration comprise entre 0,5 et 30 mmole/l de solution à examiner et le tampon en une concentration comprise entre 0,02 et 1 mole/l de solution à examiner.

3. Procédé selon la revendication 1 ou 2, caractérisé en ce que pour le dosage de la bilirubine directe la solution à examiner présente un pH compris entre 3,5 et 6,0.

4. Procédé selon l'une quelconque des revendications 1 à 3, caractérisé en ce qu'on met en œuvre en outre une substance agissant comme tiers-solvant.

5. Procédé selon la revendication 6, caractérisé en ce que la concentration de la substance agissant comme tiers-solvant dans la solution à examiner est comprise entre 0,1 et 4 moles/l pour le dosage de la bilirubine totale et entre 0,01 et 0,1 mole/l pour le dosage de la bilirubine directe.

6. Agent pour le dosage de la bilirubine directe et de la bilirubine totale dans des liquides physiologiques suivant un procédé selon les revendications 1 à 5, caractérisé en ce qu'il comporte un système tampon actif entre les pH 3,5 et 8 et un sel de diazonium de formule générale I :

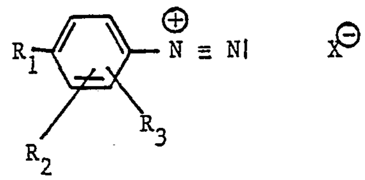

(I)

dans laquelle

$R_1$ est de l'hydrogène, de l'halogène, un groupe alkyle inférieur, alcoxy inférieur ou benzoylamino,

$R_2$ est de l'hydrogène, un groupe alkyle inférieur ou alcoxy inférieur,

$R_3$ est de l'hydrogène, de l'halogène, un groupe alkyle inférieur ou alcoxy inférieur, $R_1$ et $R_3$ ne pouvant pas être en même temps de l'halogène, et

X est un anion formant avec le cation diazonique un sel stable au stockage.

7. Agent selon la revendication 6, caractérisé en ce que le sel de diazonium et le tampon sont présents en une concentration permettant d'obtenir dans le liquide à examiner pour le dosage de la bilirubine directe et de la bilirubine totale une concentration finale comprise entre 0,5 et 30 mmoles/l pour le sel de diazonium et entre 0,02 et 1 mole/l pour le tampon.

8. Agent selon la revendication 6 ou 7, caractérisé en ce qu'il comporte en outre une substance agissant comme tiers-solvant.

9. Agent selon la revendication 8, caractérisé en ce que la substance agissant comme tiers-solvant est présente en une concentration permettant d'atteindre dans la solution à examiner une concentration finale entre 0,1 et 4 moles/l pour le dosage de la bilirubine totale et entre 0,01 et 0,1 mole/l pour le dosage de la bilirubine directe.